# EUROPEAN PATENT APPLICATION

(11) **EP 3 756 671 A1**
(43) Date of publication of application: **30.12.2020**
(21) Application number: 19754032.1
(22) Date of filing: 18.02.2019
(51) Int. Cl.: A61K 31/513, A61P 1/00, A61P 1/04, A61P 29/00

(54) **THERAPEUTIC AGENT FOR INFLAMMATORY BOWEL DISEASE**

(30) Priority: 19.02.2018 JP 2018026617
(71) Applicant: Daiichi Sankyo Company, Limited, Tokyo 103-8426 (JP)
(72) Inventor: TOYOSHIMA, Yuki, Tokyo 103-8426 (JP)
(74) Representative: Fairbairn, Angus Chisholm
(86) International application number: PCT/JP2019/005728
(87) International publication number: WO 2019/160130

(57) **Abstract**

The present invention aims to provide a pharmaceutical drug that contains a compound having an excellent therapeutic effect on inflammatory bowel disease. A therapeutic agent for inflammatory bowel disease, containing as an active ingredient a compound represented by formula (I), wherein R¹ represents a hydroxy C₁-C₆ alkyl group, a C₂-C₇ alkanoyl group, a C₂-C₇ alkanoyl C₁-C₆ alkyl group, a (C₁-C₆ alkoxy) carbonyl group, a (C₁-C₆ alkoxy) carbonyl C₁-C₆ alkyl group, a carboxy group or a carboxy C₁-C₆ alkyl group, or a pharmacologically acceptable salt thereof.

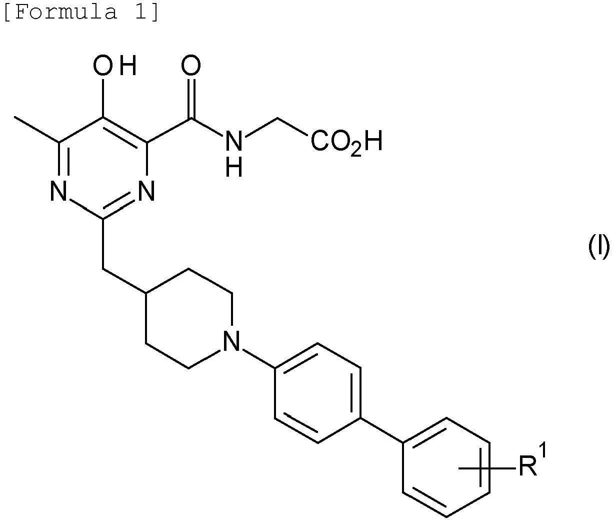

## Description

### Technical Field

The present invention relates to a therapeutic agent for inflammatory bowel disease containing a 5-hydroxypyrimidine-4-carboxamide derivative as an active ingredient.

### Background Art

Inflammatory bowel disease is a disease causing chronic inflammation or ulceration of the mucous membranes of the large and small intestines, and ulcerative colitis and Crohn's disease are representative diseases. Ulcerative colitis is a non-specific inflammatory disease of unknown cause and mainly produces erosion and ulceration of the mucous membranes from the rectum to the large intestine. Crohn's disease is a disease of unknown cause which develops discontinuous chronic granulomatous inflammation in the entire digestive tract from the oral cavity to the anus.

In addition, non-infectious colitis which is an inflammatory bowel disease in a broad sense and exhibits pathological findings on the mucous membrane of the large intestine includes lymphocytic colitis, Behcet's disease, diversion colitis, diverticular colitis, eosinophilic colitis, ischemic colitis and radiation colitis (Non

### Patent Literature 1).

Although drugs such as immunosuppressive drugs, steroids, salazosulfapyridine, or mesalazine are used for inflammatory bowel disease, they remain imperfect in terms of efficacy and safety.

On the other hand, 5-hydroxypyrimidine-4-carboxamide derivatives have excellent erythropoietin (hereinafter referred to as EPO) production enhancing activity, and are known to be effective in treating diseases caused by a decrease in EPO (Patent Literatures 1 and 2). However, it is not known that 5-hydroxypyrimidine-4-carboxamide derivatives have a therapeutic effect on inflammatory bowel disease.

### Citation List

### Patent Literature

Patent Literature 1: International Publication No. WO 2011/049126
Patent Literature 2: International Publication No. WO 2013/147214

### Non Patent Literature

Non Patent Literature 1: Nature Clinical Practice, Gastroenterology & Hepatology. 2008, 5, p.28-39

### Summary of Invention

### Technical Problem

The present invention aims to provide a pharmaceutical drug that contains a compound having an excellent therapeutic effect on inflammatory bowel disease.

### Solution to Problem

After conducting intensive studies in order to solve the above problem, the present inventors have found that the 5-hydroxypyrimidine-4-carboxamide compounds represented by the following general formula (I) or a pharmacologically acceptable salt thereof have an excellent therapeutic effect on inflammatory bowel disease, and completed the present invention.

Specifically, the present invention is:
(1) a therapeutic agent for inflammatory bowel disease, containing as an active ingredient a compound represented by the general formula (I) 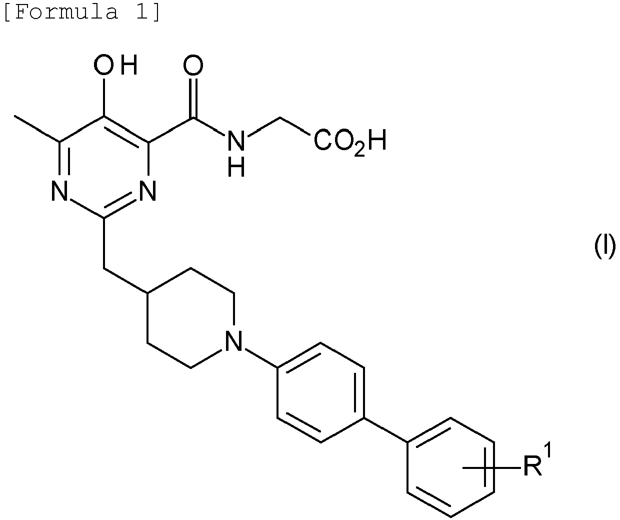
   wherein R¹ represents a hydroxy C₁-C₆ alkyl group, a C₂-C₇ alkanoyl group, a C₂-C₇ alkanoyl C₁-C₆ alkyl group, a (C₁-C₆ alkoxy) carbonyl group, a (C₁-C₆ alkoxy) carbonyl C₁-C₆ alkyl group, a carboxy group or a carboxy C₁-C₆ alkyl group,
   or a pharmacologically acceptable salt thereof;
(2) the therapeutic agent for inflammatory bowel disease according to (1), wherein R¹ is a carboxy group, a hydroxymethyl group, a 1-hydroxyethyl group, a 2-hydroxyethyl group, a 2-hydroxypropyl group, a 3-hydroxypropyl group, a 2-hydroxybutyl group, an acetyl group, a methoxycarbonyl group, an ethoxycarbonyl group, a 2-oxopropyl group, a 2-oxobutyl group, a 3-oxobutyl group, a 2-oxopentyl group, a methoxycarbonylmethyl group, or a carboxymethyl group;
(3) the therapeutic agent for inflammatory bowel disease according to (1), wherein R¹ is a carboxy group, a hydroxymethyl group, a 1-hydroxyethyl group, a 2-hydroxyethyl group, a 2-hydroxypropyl group, a 3-hydroxypropyl group, a 2-hydroxybutyl group, a 2-oxopropyl group, a 2-oxobutyl group, a 3-oxobutyl group, or a 2-oxopentyl group;
(4) the therapeutic agent for inflammatory bowel disease according to (1), wherein the compound represented by the general formula (I) is a compound selected from the group consisting of:
   ({[5-hydroxy-2-({1-[4'-(hydroxymethyl)biphenyl-4-yl]piperidin-4-yl}methyl)-6-methylpyrimidin-4-yl]carbonyl}amino)acetic acid,
   ({[5-hydroxy-2-({1-[4'-(2-hydroxypropyl)biphenyl-4-yl]piperidin-4-yl}methyl)-6-methylpyrimidin-4-yl]carbonyl}amino)acetic acid,
   [({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid,
   [({5-hydroxy-2-[(1-{4'-[(2R)-2-hydroxypropyl]biphenyl-4-yl}piperidin-4-yl)methyl]-6-methylpyrimidin-4-yl}carbonyl)amino]acetic acid,
   ({[5-hydroxy-6-methyl-2-({1-[4'-(2-oxopropyl)biphenyl-4-yl]piperidin-4-yl}methyl)pyrimidin-4-yl]carbonyl}amino)acetic acid, and
   4'-[4-({4-[(carboxymethyl)carbamoyl]-5-hydroxy-6-methylpyrimidin-2-yl}methyl)piperidin-1-yl]biphenyl-4-carboxylic acid;
(5) the therapeutic agent for inflammatory bowel disease according to (1), wherein the compound represented by the general formula (I) is [({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid;
(6) a method for treating inflammatory bowel disease by administering the therapeutic agent for inflammatory bowel disease according to any one of (1) to (5);
(7) a method for treating inflammatory bowel disease by administering the therapeutic agent for inflammatory bowel disease according to any one of (1) to (5) and any other agent;
(8) the method according to (6) or (7), wherein the inflammatory bowel disease is ulcerative colitis, Crohn's disease, lymphocytic colitis, Behcet's disease, diversion colitis, diverticular colitis, eosinophilic colitis, ischemic colitis, or radiation colitis;
(9) the method according to (6) or (7), wherein the inflammatory bowel disease is ulcerative colitis; and
(10) the method according to (6) or (7), wherein the inflammatory bowel disease is Crohn's disease.

### Advantageous Effects of Invention

The compound (I) of the present invention is useful as an active ingredient for treating inflammatory bowel disease.

In the present invention, "therapeutic agent for inflammatory bowel disease" refers to a drug having a therapeutic effect on ulcerative colitis, Crohn's disease, lymphocytic colitis, Behcet's disease, diversion colitis, diverticular colitis, eosinophilic colitis, ischemic colitis, or radiation colitis.

### Brief Description of Drawings

[Figure 1] Figure 1 is a graph showing the change over time in body weight of a compound A administered group, an untreated group and a negative control group in colitis model mice.
[Figure 2] Figure 2 is a graph showing the amount of change in body weight on Day 8 of a compound A administered group, an untreated group and a negative control group in colitis model mice.
[Figure 3] Figure 3 is a graph showing the intestinal tract length on Day 8 of a compound A administered group, an untreated group and a negative control group in colitis model mice.
[Figure 4] Figure 4 is a graph showing the diarrhea score on Day 8 of a compound A administered group, an untreated group and a negative control group in colitis model mice.

### Description of Embodiments

The therapeutic agent for inflammatory bowel disease of the present invention contains the compound (I) of the present invention or a pharmacologically acceptable salt thereof as an active ingredient.

Hereinafter, the substituents in the compound (I) of the present invention will be described.

The "C₁-C₆ alkyl group" refers to a linear or branched alkyl group having 1 to 6 carbon atoms. Examples thereof include a methyl group, an ethyl group, a propyl group, an isopropyl group, a butyl group, a sec-butyl group, a tert-butyl group, a pentyl group, an isopentyl group, a 2-methylbutyl group, a neopentyl group, a 1-ethylpropyl group, a hexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, and a 2-ethylbutyl group.

The "hydroxy C₁-C₆ alkyl group" in the definition of R¹ refers to a group in which one or more hydrogen atoms (suitably, one or two hydrogen atoms) of the "C₁-C₆ alkyl group" are each replaced by a hydroxyl group. Examples thereof include a hydroxymethyl group, a 1-hydroxyethyl group, a 2-hydroxyethyl group, a 1-hydroxypropyl group, a 2-hydroxypropyl group, a 3-hydroxypropyl group, a 2-hydroxy-1,1-dimethylethyl group, a 2-hydroxybutyl group and a 2-hydroxypentyl group. The hydroxy C₁-C₆ alkyl group is preferably a hydroxymethyl group, a 1-hydroxyethyl group, a 2-hydroxyethyl group, a 2-hydroxypropyl group, a 3-hydroxypropyl group, or a 2-hydroxybutyl group, and more preferably a hydroxymethyl group or a 2-hydroxypropyl group.

The "C₂-C₇ alkanoyl group" in the definition of R¹ refers to a group in which the above "C₁-C₆ alkyl group" is bonded to a carbonyl group. Examples thereof include an acetyl group, a propionyl group, a butyryl group, an isobutyryl group, a pentanoyl group, a pivaloyl group, a valeryl group, an isovaleryl group, a hexanoyl group, and a heptanoyl group. The C₂-C₇ alkanoyl group is preferably an acetyl group.

The "C₂-C₇ alkanoyl C₁-C₆ alkyl group" in the definition of R¹ refers to a group in which one hydrogen atom of the above "C₁-C₆ alkyl group" is replaced by the above "C₂-C₇ alkanoyl group". Examples thereof include a 2-oxopropyl group, a 2-oxobutyl group, a 3-oxobutyl group, a 2-oxopentyl group, a 3-oxopentyl group, and a 4-oxopentyl group. The C₂-C₇ alkanoyl C₁-C₆ alkyl group is preferably a 2-oxopropyl group, a 2-oxobutyl group, a 3-oxobutyl group, or a 2-oxopentyl group, more preferably a 2-oxopropyl group.

The "C₁-C₆ alkoxy group" in the definition of R¹ refers to a group in which the above "C₁-C₆ alkyl group" is bonded to an oxygen atom. Examples thereof include a methoxy group, an ethoxy group, an n-propoxy group, an n-butoxy group, an s-butoxy group, a tert-butoxy group, and an n-pentoxy group.

The "(C₁-C₆ alkoxy) carbonyl group" in the definition of R¹ refers to a group in which the above "C₁-C₆ alkoxy group" is bonded to a carbonyl group. Examples thereof include a methoxycarbonyl group, an ethoxycarbonyl group, an n-propoxycarbonyl group, and an n-butoxycarbonyl group. The (C₁-C₆ alkoxy) carbonyl group is preferably a methoxycarbonyl group or an ethoxycarbonyl group.

The " (C₁-C₆ alkoxy) carbonyl C₁-C₆ alkyl group" in the definition of R¹ refers to a group in which the above "(C₁-C₆ alkoxy) carbonyl group" is bonded to the above "C₁-C₆ alkyl group". Examples thereof include a methoxycarbonylmethyl group, a methoxycarbonylethyl group, an ethoxycarbonylmethyl group, an ethoxycarbonylethyl group, an n-propoxycarbonylmethyl group, an n-propoxycarbonylethyl group, an n-butoxycarbonylmethyl group, and an n-butoxycarbonylethyl group. The (C₁-C₆ alkoxy) carbonyl C₁-C₆ alkyl group is preferably a methoxycarbonylmethyl group.

The "carboxy C₁-C₆ alkyl group" in the definition of R¹ refers to a group in which the carboxy group is bonded to the above "C₁-C₆ alkyl group". Examples thereof include a carboxymethyl group, a 1-carboxyethyl group, a 2-carboxyethyl group, a 1-carboxypropyl group, a 2-carboxypropyl group, a 3-carboxypropyl group, a 2-carboxy-1,1-dimethylethyl group, a 2-carboxybutyl group, and a 2-carboxypentyl group. The carboxy C₁-C₆ alkyl group is preferably a carboxymethyl group.

In the present invention, R¹ preferably represents a carboxy group, a hydroxymethyl group, a 1-hydroxyethyl group, a 2-hydroxyethyl group, a 2-hydroxypropyl group, a 3-hydroxypropyl group, a 2-hydroxybutyl group, an acetyl group, a methoxycarbonyl group, an ethoxycarbonyl group, a 2-oxopropyl group, a 2-oxobutyl group, a 3-oxobutyl group, a 2-oxopentyl group, a methoxycarbonylmethyl group, or a carboxymethyl group, more preferably a carboxy group, a hydroxymethyl group, a 1-hydroxyethyl group, a 2-hydroxyethyl group, a 2-hydroxypropyl group, a 3-hydroxypropyl group, a 2-hydroxybutyl group, a methoxycarbonyl group, an ethoxycarbonyl group, a 2-oxopropyl group, a 2-oxobutyl group, a 3-oxobutyl group, or a 2-oxopentyl group, and further preferably a carboxy group, a hydroxymethyl group, a 2-hydroxypropyl group, or a 2-oxopropyl group.

The compound (I) of the present invention or the pharmacologically acceptable salt thereof is preferably one selected from the following compounds or the pharmacologically acceptable salts thereof:
({[5-hydroxy-2-({1-[4'-(hydroxymethyl)biphenyl-4-yl]piperidin-4-yl}methyl)-6-methylpyrimidin-4-yl]carbonyl}amino)acetic acid,
({[5-hydroxy-2-({1-[4'-(2-hydroxypropyl)biphenyl-4-yl]piperidin-4-yl}methyl)-6-methylpyrimidin-4-yl]carbonyl}amino)acetic acid,
[({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid,
[({5-hydroxy-2-[(1-{4'-[(2R)-2-hydroxypropyl]biphenyl-4-yl}piperidin-4-yl)methyl]-6-methylpyrimidin-4-yl}carbonyl)amino]acetic acid,
({[5-hydroxy-6-methyl-2-({1-[4'-(2-oxopropyl)biphenyl-4-yl]piperidin-4-yl}methyl)pyrimidin-4-yl]carbonyl}amino)acetic acid, or
4'-[4-({4-[(carboxymethyl)carbamoyl]-5-hydroxy-6-methylpyrimidin-2-yl}methyl)piperidin-1-yl]biphenyl-4-carboxylic acid;
more preferably, [({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid, ({[5-hydroxy-6-methyl-2-({1-[4'-(2-oxopropyl)biphenyl-4-yl]piperidin-4-yl}methyl)pyrimidin-4-yl]carbonyl}amino)acetic acid, or 4'-[4-({4-[(carboxymethyl)carbamoyl]-5-hydroxy-6-methylpyrimidin-2-yl}methyl)piperidin-1-yl]biphenyl-4-carboxylic acid; and further preferably [({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid.

When the compound (I) of the present invention has an asymmetric carbon atom, optical isomers may exist. The present invention encompasses separated forms of these isomers (for example, enantiomers or diastereomers) and mixtures thereof (for example, racemic or diastereomeric mixtures).

When the compound (I) of the present invention has a basic group such as an amino group, a pharmacologically acceptable acid addition salt can be formed, as desired. Examples of such acid addition salts include hydrohalides such as hydrofluorides, hydrochlorides, hydrobromides, and hydroiodides; inorganic acid salts such as nitrates, perchlorates, sulfates, and phosphates; lower alkane sulfonates such as methanesulfonates, trifluoromethanesulfonates and ethanesulfonates; aryl sulfonates such as benzenesulfonates and p-toluenesulfonates; organic acid salts such as acetic acid, malic acid, fumarates, succinates, citrates, tartrates, oxalates, and maleates; and amino acid salts such as ornithates, glutamates, and aspartates, and it is preferably a hydrohalide or an organic acid salt.

When the compound (I) of the present invention has an acidic group such as a carboxy group, it is generally possible to form a pharmacologically acceptable base addition salt. Examples of such base addition salts include alkali metal salts such as sodium salts, potassium salts, and lithium salts; alkaline earth metal salts such as calcium salts and magnesium salts; inorganic salts such as ammonium salts; and organic amine salts such as dibenzylamine salts, morpholine salts, phenylglycine alkyl ester salts, ethylenediamine salts, N-methylglucamine salts, diethylamine salts, triethylamine salts, cyclohexylamine salts, dicyclohexylamine salts, N,N'-dibenzylethylenediamine salts, diethanolamine salts, N-benzyl-N-(2-phenylethoxy)amine salts, piperazine salts, tetramethylammonium salts and

### tris(hydroxymethyl)aminomethane salts.

The compound (I) of the present invention exists as a non-solvate or a solvate. The solvate is not particularly limited as long as it is pharmacologically acceptable, but specifically, a hydrate, an ethanol solvate, or the like is preferable.

The compound (I) of the present invention can be produced, for example, by the method described in WO 2011/049126 or WO 2013/147214.

The method for administering the therapeutic agent for inflammatory bowel disease of the present invention is not particularly limited, and it is administered orally or parenterally by a method according to the various preparation forms, the patient's age, gender and other conditions, the severity of the disease, and the like. Examples of preparation forms for oral administration include tablets, pills, capsules, granules, powders, suspensions, emulsions, and liquids, and examples for parenteral administration include local administration agents, injections, transdermal agents, suppositories, nasal agents, inhalants, and enemas.

The dose and the number of administrations of the therapeutic agent for inflammatory bowel disease of the present invention are appropriately selected according to the usage, the patient's age, gender and other conditions, and the severity of the disease. For example, the dose is usually 0.01 mg/kg to 100 mg/kg per administration for an adult, and the number of administrations is usually once to six times a day. The content of the active ingredient in the preparation is usually 0.0001 to 30 weight% (the upper limit is suitably 10 weight%, more suitably 1 weight%), further preferably 0.001 to 0.1 weight%, and particularly preferably 0.01 to 0.03 weight%. If necessary, the preparation can be formulated with additives such as absorption promoters, pH adjusters, preservatives, flavoring agents, dispersants, wetting agents, stabilizers, preserving agents, suspending agents, and surfactants.

The therapeutic agent for inflammatory bowel disease of the present invention can be administered together with immunosuppressive drugs, steroids, salazosulfapyridine or mesalazine, anti-TNFα preparations, or the like.

### Examples

Hereinafter, the present invention will be further described in detail by way of the examples and test examples, but the scope of the present invention is not limited thereto.

### (Example 1)

[({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid (Compound A)

Compound A was prepared according to the method of Example 45 in WO 2011/049126.

(Test Example 1) Efficacy of Compound A in model of dextran sulfate sodium-induced colitis

[Model preparation and compound administration method]

A colitis model was prepared by allowing 7-week-old male C57BL/6J mice to freely drink a 1.5% aqueous solution of dextran sulfate sodium (hereinafter, DSS) dissolved in pure water purified through a Milli-Q (registered trademark, Merck Millipore) filter (hereinafter, referred to as Milli-Q water). For the control group, only Milli-Q water was administered. Simultaneously with the start of administration of DSS in drinking water, feed prepared by mixing Compound A (0.01%, 0.03%) with powdered FR-2 was administered to the feed administered group. For the vehicle group with feeding, and the control group, to which no DSS was administered, only powdered FR-2 was administered. The day on which the administration of DSS in drinking water and the administration of the compound were started was designated as Day 1, and the administration was continued until Day 8.

### [Test group configuration]

Control group: Milli-Q water, FR-2 (N=10)
Vehicle group: 1.5% DSS solution, FR-2 (N=10)
Compound A 0.01% group: 1.5% DSS solution, FR-2 mixed with 0.01% Compound A (N=10)
Compound A 0.03% group: 1.5% DSS solution, FR-2 mixed with 0.03% Compound A (N=10)

### [Experimental operation]

All animals were weighed in the morning from Day 1 to Day 8. On Day 8, the abdomen was opened under isoflurane anesthesia, blood was collected from the abdominal vein by an untreated syringe, the duodenum, large intestine, rectum, and anus were removed, and the length of the removed large intestine was measured with an electric caliper. The large intestine was cut open to observe the stool properties and score the degree of diarrhea. The diarrhea scores are shown in Table 1.

**(Table 1)**

| Diarrhea score | Stool properties |
|---|---|
| 0 | Normal stool |
| 1 | Loose stool (tangible stool with high water content) |
| 2 | Diarrhea stool (stool with a high water content that has lost its shape) |
| 3 | Watery stool (almost intangible liquid stool) |

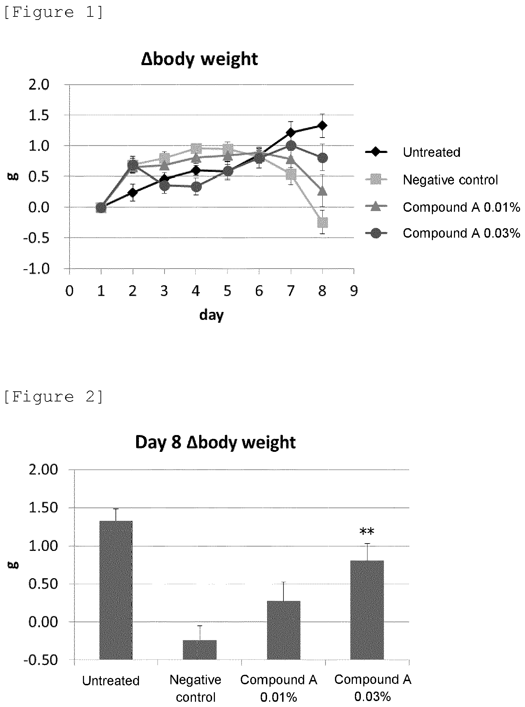

Figure 1 shows the change in body weight over days, in which the body weight on Day 1 is taken as 0, and Figure 2 shows the amount of change in body weight on Day 8. Compared with the negative control, the administration of 0.03% of Compound A in the feed showed a significant inhibitory effect on weight loss.

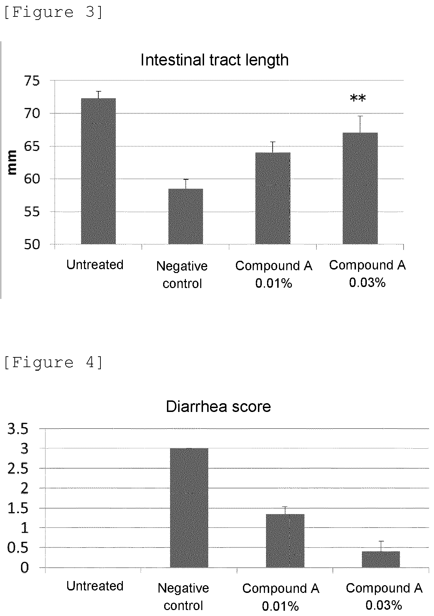

Figure 3 shows the intestinal tract length on Day 8. Compared with the negative control, the administration of 0.03% of Compound A in the feed showed a significant inhibitory effect on intestinal length shortening.

Figure 4 shows the diarrhea score on Day 8. Compared with the negative control, the compound-administered group tended to have improved stool properties.

Compound A showed an improving effect on the clinical condition in a DSS-induced colitis model. This showed that a 5-hydroxypyrimidine-4-carboxamide derivative is useful as a therapeutic agent for inflammatory bowel disease.

### Industrial Applicability

The compound (I) of the present invention is useful as a therapeutic agent for inflammatory bowel disease. Therefore, the therapeutic agent for inflammatory bowel disease of the present invention can be used for treating ulcerative colitis, Crohn's disease, lymphocytic colitis, Behcet's disease, diversion colitis, diverticular colitis, eosinophilic colitis, ischemic colitis, or radiation colitis.

## Claims

1. A therapeutic agent for inflammatory bowel disease, comprising as an active ingredient a compound represented by the general formula (I) 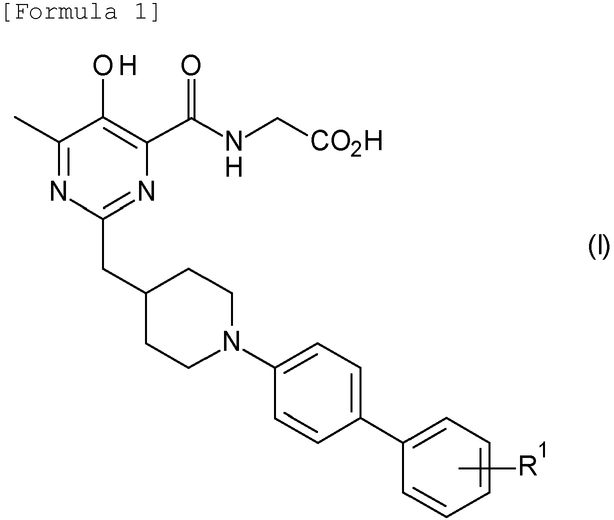 wherein R¹ represents a hydroxy C₁-C₆ alkyl group, a C₂-C₇ alkanoyl group, a C₂-C₇ alkanoyl C₁-C₆ alkyl group, a (C₁-C₆ alkoxy) carbonyl group, a (C₁-C₆ alkoxy) carbonyl C₁-C₆ alkyl group, a carboxy group or a carboxy C₁-C₆ alkyl group,
or a pharmacologically acceptable salt thereof.

2. The therapeutic agent for inflammatory bowel disease according to claim 1, wherein R¹ is a carboxy group, a hydroxymethyl group, a 1-hydroxyethyl group, a 2-hydroxyethyl group, a 2-hydroxypropyl group, a 3-hydroxypropyl group, a 2-hydroxybutyl group, an acetyl group, a methoxycarbonyl group, an ethoxycarbonyl group, a 2-oxopropyl group, a 2-oxobutyl group, a 3-oxobutyl group, a 2-oxopentyl group, a methoxycarbonylmethyl group, or a carboxymethyl group.

3. The therapeutic agent for inflammatory bowel disease according to claim 1, wherein R¹ is a carboxy group, a hydroxymethyl group, a 1-hydroxyethyl group, a 2-hydroxyethyl group, a 2-hydroxypropyl group, a 3-hydroxypropyl group, a 2-hydroxybutyl group, a 2-oxopropyl group, a 2-oxobutyl group, a 3-oxobutyl group, or a 2-oxopentyl group.

4. The therapeutic agent for inflammatory bowel disease according to claim 1, wherein the compound represented by the general formula (I) is a compound selected from the group consisting of:
({[5-hydroxy-2-({1-[4'-(hydroxymethyl)biphenyl-4-yl]piperidin-4-yl}methyl)-6-methylpyrimidin-4-yl]carbonyl}amino)acetic acid,
({[5-hydroxy-2-({1-[4'-(2-hydroxypropyl)biphenyl-4-yl]piperidin-4-yl}methyl)-6-methylpyrimidin-4-yl]carbonyl}amino)acetic acid,
[({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid,
[({5-hydroxy-2-[(1-{4'-[(2R)-2-hydroxypropyl]biphenyl-4-yl}piperidin-4-yl)methyl]-6-methylpyrimidin-4-yl}carbonyl)amino]acetic acid,
({[5-hydroxy-6-methyl-2-({1-[4'-(2-oxopropyl)biphenyl-4-yl]piperidin-4-yl}methyl)pyrimidin-4-yl]carbonyl}amino)acetic acid, and
4'-[4-({4-[(carboxymethyl)carbamoyl]-5-hydroxy-6-methylpyrimidin-2-yl}methyl)piperidin-1-yl]biphenyl-4-carboxylic acid.

5. The therapeutic agent for inflammatory bowel disease according to claim 1, wherein the compound represented by the general formula (I) is [({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid.

6. A method for treating inflammatory bowel disease by administering the therapeutic agent for inflammatory bowel disease according to any one of claims 1 to 5.

7. A method for treating inflammatory bowel disease by administering the therapeutic agent for inflammatory bowel disease according to any one of claims 1 to 5 and any other agent.

8. The method according to claim 6 or 7, wherein the inflammatory bowel disease is ulcerative colitis, Crohn's disease, lymphocytic colitis, Behcet's disease, diversion colitis, diverticular colitis, eosinophilic colitis, ischemic colitis, or radiation colitis.

9. The method according to claim 6 or 7, wherein the inflammatory bowel disease is ulcerative colitis.

10. The method according to claim 6 or 7, wherein the inflammatory bowel disease is Crohn's disease.
